# FASCICULE DE BREVET EUROPEEN

(11) **EP 1 050 287 B1**
(45) Date de publication et mention de la délivrance du brevet: **25.07.2007**
(21) Numéro de dépôt: 99401122.9
(22) Date de dépôt: 07.05.1999
(51) Int. Cl.: A61F 13/00, A61F 5/01

(54) **Article medical de contention**
Medizinischer Kompressionsartikel
Medical compression article

(43) Date de publication de la demande: 08.11.2000
(73) Titulaire: THUASNE Société Anonyme, 92307 Levallois-Perret Cedex (FR)
(72) Inventeur: Picolet, Jean-Pierre, 42270 Saint Peiest en Jarez (FR); Courtet, François, 43330 Saint Galmier (FR)
(74) Mandataire: Eidelsberg, Victor Albert

(56) Documents cités:
- EP-A- 0 619 103
- DE-A- 3 902 434
- FR-A- 2 642 303
- FR-A- 2 772 596
- US-A- 4 111 194
- US-A- 5 090 404
- US-A- 5 407 422

## Description

La présente invention est relative aux articles médicaux de contention, notamment pour le corps humain.

Ces articles comportent un panneau de matière souple, en général textile, destiné à entourer une partie au moins d'un membre et au moins un élément de serrage du panneau sur ce membre.

Ces articles donnent en général satisfaction, mais ils présentent parfois un inconvénient qui réside en ce que, par l'effet de la gravité, des mouvements du membre ou du corps et/ou de la forme anatomique de ce membre, ils ont tendance à glisser par rapport à leur position de pose, ce qui leur fait perdre leur efficacité et oblige à une nouvelle mise en place.

Le document DE 3902 434 concerne une genouillère comprenant de la matière anti-glissement sur la partie inférieure et sur la partie supérieure.

Le document US-A-4.111.194 prévoit de disposer de la matière anti-glissement sur toute la longueur du panneau et des deux côtés. Il en résulte un coût en matière élevée et en outre l'aération de la peau du patient est gênée par le fait que cette matière s'étend sur toute la longueur et des deux côtés.

Le document EP-0.619.103 concerne un harnais pour le dos, dans lequel la matière anti-glissement se trouve des deux côtés et en s'étendant du bas jusqu'en haut.

Dans le cas du brevet français 2.642.303, le matériau anti-glissement est également déposé sur toute l'étendue de l'article et des deux côtés, article qui d'ailleurs dans le cas de son application à un genou, n'a pas de plaque, puisqu'il s'agit uniquement d'un ruban.

Le document US-A-5.090.404 (KALLASSY) concerne une attelle de cheville dans lequel également le matériau anti-glissement s'étend sur toute la longueur en hauteur de l'attelle, et des deux côtés.

Enfin le document US-A-5.507.422 (MATTHIJS) concerne un harnais pour le dos où la matière anti-glissement se trouve des deux côtés et en s'étendant du bas jusqu'en haut.

L'invention s'applique à un article médical de contention qui est constitué par une attelle d'immobilisation du genou.

Cette attelle comporte un panneau ouvert en matière textile qui est équipé de montants métalliques rigides d'immobilisation, rectilignes ou coudés, et de sangles de serrage ou de fermeture au-dessus et au-dessous du genou, c'est-à-dire sur la cuisse et sur la jambe, par exemple à l'aide de fermetures du type auto-agrippant.

Suivant l'invention on obtient une meilleure aération avec moins de produit anti-glissement et ce avec malgré tout un maintien aussi bon que dans le cas des attelles de l'art antérieur.

Conformément à l'invention, pour éviter ainsi un glissement de l'article médical par rapport au membre à contenir, pour l'une ou l'autre des raisons indiquées ci-dessus tout en assurant une meilleure aération de l'utilisateur et un coût moindre en matière (nom du matériau ant-glissement), l'article médical est tel que défini à la revendication 1.

Suivant les perfectionnements définis aux revendications 2 à 4, on obtient un maintien particulièrement efficace et stable de l'article sur le membre à contenir.

Ainsi, dans l'article selon l'invention, on assure non seulement la fonction habituelle normale de contention, mais également la fonction de retenue par friction de l'article sur le membre, ce qui évite les inconvénients ci-dessus.

La matière anti-glissement est du type caoutchouc, naturel ou synthétique, par exemple connu sous la marque Néoprène^{R}, ou encore en silicone de type anti-glissement.

Selon l'aire qu'elle occupe sur la surface intérieure du panneau, la matière anti-glissement peut être pleine, par exemple pour une aire faible, ou au contraire ajourée pour permettre à la peau de respirer, notamment dans le cas d'une aire importante.

De préférence, pour que la matière anti-glissement ait une efficacité maximum, elle est présente au moins dans une zone de recouvrement d'une partie du membre à forte courbure, notamment l'arête tibiale avant dans le cas d'une genouillère ou d'une attelle d'immobilisation du genou.

Par des essais, on est arrivé à la conclusion qu'il est préférable, dans le cas où la matière anti-glissement n'existe que localement, qu'elle soit placée à la partie inférieure du panneau.

Selon l'usage auquel l'article médical selon l'invention est destiné, le panneau peut être en une matière élastique au moins transversalement, ou en une matière non élastique, auquel cas les moyens de serrage peuvent être constitués par des sangles, notamment du type auto-agrippant.

Avantageusement, la matière anti-glissement est constituée par une pièce qui est rapportée sur la surface intérieure du panneau, cette pièce pouvant être amovible, notamment à l'aide d'une fermeture du type auto-agrippant, ce qui permet de la supprimer par exemple lors des phases de repos ou d'immobilité du patient.

On comprendra bien l'invention à la lecture du complément de description qui va suivre et en référence aux dessins annexés qui font partie de la description et dans lesquels :
Fig. 1 est une vue arrachée en élévation, du côté intérieur, montrant une attelle d'immobilisation du genou, à plat, selon un mode de réalisation préféré de l'invention ;
Fig. 2 est une vue arrachée analogue à la Fig. 1 et montrant l'attelle du côté extérieur ;
Fig. 3 montre, à plus grande échelle, une partie de l'attelle de la Fig. 1 de laquelle la pièce a été supprimée ;
Fig. 4 est, à plus grande échelle, une vue arrachée en élévation du côté extérieur de la pièce anti-glissement ;
Fig. 5 est une coupe longitudinale verticale, suivant le plan V-V de la Fig. 4, montrant la pièce anti-glissement montée, de manière amovible, sur une pièce de support elle-même fixée sur la face intérieure du panneau ; et
Fig. 6 est une vue en élévation latérale montrant une attelle d'immobilisation du genou en position d'utilisation.

La description qui va suivre sera faite dans l'application préférée, mais non limitative, de l'invention à une attelle d'immobilisation du genou, suivant deux modes de réalisation de cette attelle.

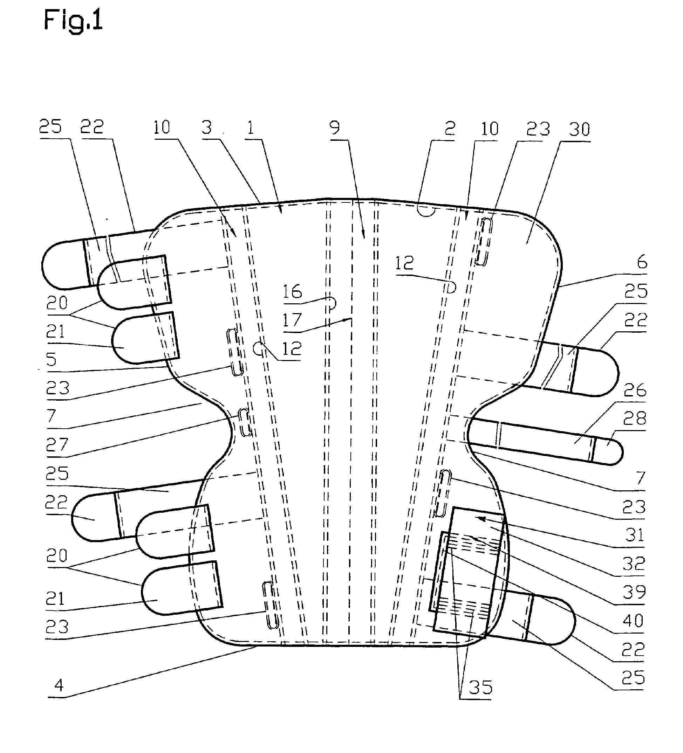

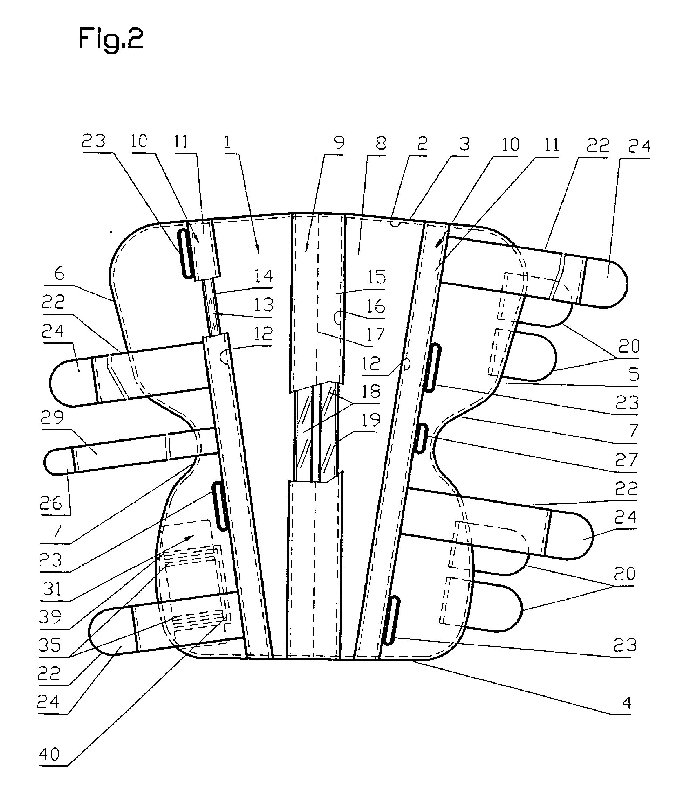

Sur les Figs. 1 et 2, on a représenté, vue en élévation du côté intérieur et extérieur, une telle attelle d'immobilisation. Cette attelle comporte, à l'état déployé à plat, un panneau textile souple 1 qui se présente sous la forme générale d'un trapèze isocèle. De manière connue, le panneau 1 est constitué par un complexe textile qui comporte par exemple une couche intérieure tricotée, une couche intermédiaire en mousse et une couche extérieure également tricotée, ces trois couches étant assujetties l'une à l'autre par une couture périphérique 2. Le contour trapézoïdal est constitué par une grande base supérieure 3 sensiblement rectiligne, une petite base inférieure 4 également sensiblement rectiligne, et deux côtés inclinés 5, 6 sensiblement rectilignes et présentant une échancrure centrale 7 destinée à entourer la zone rotulienne.

Sur sa face extérieure 8, le panneau 1 comporte trois goussets, à savoir un double gousset longitudinal central 9, dans l'axe de symétrie du trapèze, et deux goussets latéraux 10.

Chaque gousset latéral 10 est défini par une bande étroite 11, par exemple en matière textile, qui est cousue, par deux lignes latérales de couture 12, sur la face extérieure 8 du panneau 1, le gousset étant fermé à ses deux extrémités par la couture périphérique 2. Chaque gousset 10 emprisonne avec un faible jeu un montant rigide d'immobilisation 13, en général métallique, un coussin d'amortissement 14 étant disposé entre le montant 13 et la surface 8 du panneau 1.

Le double gousset central 9 est défini par une bande 15 en matière textile qui est fixée sur le panneau 1 par deux coutures 16 le long de ses bords et par une couture centrale 17, de manière à définir deux poches. Les extrémités du gousset 9 sont fermées par la couture périphérique 2 du panneau 1. De manière analogue aux goussets latéraux 10, le double gousset central 9 emprisonne avec un faible jeu deux montants rigides espacés 18, par exemple métalliques, avec interposition d'un coussin unique d'amortissement 19 qui est cousu le long de la ligne de couture centrale 17.

En variante, les montants 13 et 18 peuvent être amovibles ; pour cela, on peut par exemple pratiquer sur chaque bande 11 et 15 une fente transversale, à proximité d'une extrémité des goussets.

Les goussets latéraux 10 convergent vers la petite base 4 pour épouser la forme immobilisée du membre lors de la pose.

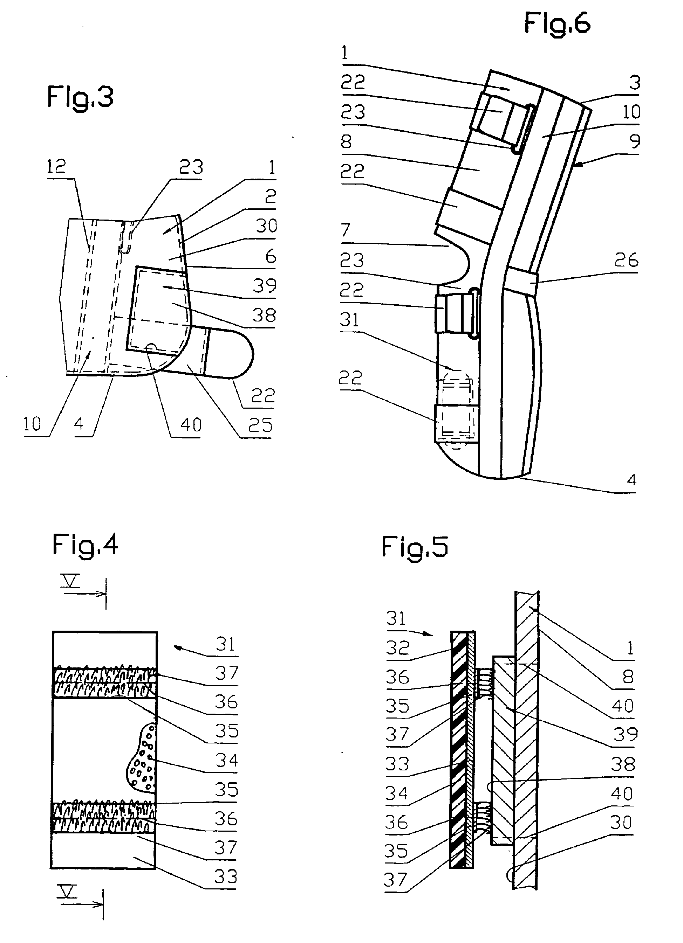

Dans l'exemple des Figs. 1 et 2, les montants 13 sont rectilignes, ce qui correspond à une position totalement tendue du membre, tandis que, dans l'attelle de la Fig. 6, ils sont coudés dans la zone du genou pour présenter, de part et d'autre de cette zone, deux parties rectilignes inclinées l'une par rapport à l'autre, ce qui correspond à une position d'immobilisation partiellement fléchie du membre. Quant aux montants arrière 18, ils sont galbés pour épouser la forme anatomique du membre.

Le panneau 1 est également équipé de moyens de fermeture ou de serrage qui permettent la mise en place de l'attelle sur le membre. Ces moyens comportent tout d'abord par exemple quatre courtes languettes 20 qui font saillie à partir du bord latéral 5 et dont la face intérieure 21 portant des crochets peut venir coopérer, dans une liaison d'accrochage séparable du type auto-agrippant, avec la surface extérieure complémentaire 8 du panneau 1 qui, à cet effet, présente une structure bouclée, pelucheuse ou analogue. Les languettes 20 servent à pré-positionner l'attelle sur le membre. Les moyens de serrage comportent également par exemple quatre longues sangles transversales 22 alternées qui partent d'un gousset latéral 10 et qui sont destinées à s'engager dans une boucle opposée 23, située latéralement dans la zone de l'autre gousset latéral 10. Les sangles 22 et les boucles 23 sont par exemple immobilisées par les coutures extérieures 12 des goussets latéraux. Chaque sangle 22 est destinée à se refermer ou se replier sur elle-même après passage dans la boucle associée 23, dans une liaison d'accrochage séparable du type auto-agrippant. A cet effet, en considérant l'attelle à plat, comme représenté sur les Figs. 1 et 2, l'extrémité libre de chaque sangle 22 présente une face extérieure accrochante 24, de préférence du type à crochets, tandis que la surface intérieure 25 du reste de la sangle présente une structure accrochante complémentaire, par exemple bouclée, pelucheuse ou analogue. Les sangles 22 font saillie latéralement à l'extérieur des bords latéraux inclinés 5, 6 du panneau 1, et elles sont destinées à passer devant le membre. Les éléments de serrage ci-dessus peuvent avantageusement être complétés par une sangle centrale additionnelle 26, du même type que les sangles 22, qui part d'un gousset latéral 10 en faisant saillie latéralement d'un bord du panneau, parallèlement aux sangles adjacentes 22, pour venir coopérer avec une boucle centrale associée 27 portée par l'autre gousset latéral 10, de manière analogue aux boucles adjacentes 23. La sangle 26 est située dans la zone de l'échancrure correspondante 7 pour passer derrière le membre, dans la zone du creux poplité de manière à plaquer la partie intermédiaire des montants centraux 18 contre cette zone. Du fait que la sangle 26 passe derrière le membre, et non pas devant celui-ci comme les sangles 22, et qu'elle est montée sur le bord extérieur du gousset latéral 10, c'est la surface intérieure 28 de son extrémité libre qui porte des crochets, tandis que la surface extérieure 29 de cette sangle porte des boucles ou analogues.

Les notions de surface intérieure et de surface extérieure pour les sangles 22 sont à considérer pour la position à plat de l'attelle. Il va de soi que, du fait que ces sangles passent devant le membre et sont repliées sur elles-mêmes à 180° après passage dans les boucles 23, ces notions sont à inverser lorsque l'on considère l'attelle en position sur le membre, cette inversion n'étant pas applicable à la sangle intermédiaire 26 puisque celle-ci passe derrière le membre et que, par rapport à sa position représentée sur les Figs. 1 et 2, elle est repliée deux fois à 180° sur elle-même pour prendre sa forme et sa position finales de pose. En variante, la sangle 26 peut être montée sur le bord intérieur du gousset latéral 10, auquel cas les notions de surface intérieure et extérieure sont également à inverser pour elle.

Lorsque l'attelle est mise en place sur le membre, elle est refermée sur celui-ci de l'arrière vers l'avant, les montants centraux 18 étant dans le plan sagittal et en position arrière sur le membre, tandis que les montants latéraux 13 sont situés sur les côtés. Du fait que les languettes 20 sont montées sur le côté 5 du trapèze, c'est l'autre bordure, correspondant au côté 6 du trapèze, qui est d'abord placée sur le membre, l'autre bordure, correspondant au côté 5 du trapèze, étant ensuite placée par-dessus. Les languettes 20 servent à pré-positionner l'attelle sur le membre, par coopération de leur surface accrochante 21 avec la surface extérieure 8 du panneau 1. Les quatre sangles 22 sont ensuite passées par-devant le membre, elles sont enfilées dans les boucles 23 et rabattues sur elles-mêmes à 180°, suivant une tension voulue, pour serrer de manière appropriée l'attelle sur le membre, la retenue se faisant par la coopération de la surface extrême accrochante 24 avec la surface opposée 25. La sangle intermédiaire supplémentaire 26 est ensuite mise en place par derrière le membre, elle est passée dans la boucle 27 et rabattue sur elle-même à 180° pour que sa surface extrême accrochante 28 vienne coopérer avec la surface opposée intermédiaire complémentaire 29. Comme indiqué précédemment, cette sangle additionnelle 26 a principalement pour fonction de maintenir les montants centraux 18 contre le creux poplité du genou.

Conformément à une caractéristique de l'invention, pour éviter que l'attelle ne glisse vers le bas, pour les raisons ci-dessus, une partie au moins de la surface intérieure 30 du panneau 1, c'est-à-dire la surface de celui-ci qui est tournée vers le membre et qui est en contact direct ou indirect avec lui, est en une matière anti-glissement qui présente un fort coefficient de frottement.

Suivant le mode de réalisation préféré de l'invention représenté sur les Figs. 1 à 5, on fait porter par la surface intérieure 30 du panneau, à la partie inférieure de celui-ci et seulement d'un seul côté, une pièce rapportée amovible 31 dont la surface intérieure 32 présente de telles propriétés d'anti-glissement. La pièce 31 s'étend par exemple depuis le bord 6 du trapèze jusqu'au voisinage de la zone du gousset latéral adjacent 10.

La pièce rapportée 31 est formée par un complexe textile constitué par exemple d'une couche arrière tricotée de support 33 sur laquelle est collée une couche 34 de matière anti-glissement, par exemple du type caoutchouc, naturel ou synthétique, notamment en Néoprène^{R}, ou en silicone du type anti-glissement.

La pièce rapportée 31 est montée sur la face intérieure 30 du panneau 1 par l'intermédiaire de deux fermetures du type auto-agrippant. Chaque fermeture comporte une bande transversale 35 qui est cousue en 36 sur la face arrière de la couche de support 33 et dont les éléments d'accrochage 37, par exemple des crochets, sont destinés à coopérer avec la surface complémentaire 38 d'une pièce textile 39 cousue en 40 sur la face intérieure 30 du panneau 1.

Dans le cas décrit ici d'une attelle d'immobilisation du genou, la matière anti-glissement est située dans une zone de l'attelle qui vient recouvrir au moins une partie du membre à forte courbure, c'est-à-dire à faible rayon de courbure, pour agir ainsi dans une zone où, pour une tension transversale donnée de l'attelle, la pression de l'attelle sur le membre est importante du fait que cette pression est inversement proportionnelle au rayon de courbure. On sait en effet que la pression en un point est proportionnelle à la tension et inversement proportionnelle au rayon de courbure. Dans le cas décrit ici, la pièce rapportée 31 est donc située au niveau de l'arête tibiale avant, et de préférence à la partie inférieure de l'attelle car c'est pour cette position, comme l'ont montré des essais, que l'effet de retenue est le plus important.

Il est à noter qu'une même attelle peut être placée indifféremment sur le membre inférieur droit ou gauche.

Comme on l'a déjà évoqué précédemment, les montants rigides incorporés à l'attelle peuvent être rectilignes, comme dans les Figs. 1 et 2, ou bien légèrement coudés, comme représenté sur la Fig. 6, selon que le membre est à immobiliser en position tendue ou légèrement fléchie.

En fonction notamment de l'importance de la surface du corps qu'elle recouvre, la matière anti-glissement constituée ici par la pièce rapportée 31, peut être pleine ou ajourée, dans ce dernier cas pour permettre une ventilation de la peau.

Dans la description ci-dessus de deux modes de réalisation appliqués à une attelle d'immobilisation, le panneau 1 est en une matière non élastique ; toutefois, on peut prévoir, dans cette application particulière ou dans d'autres applications, que le panneau, qu'il soit ouvert ou en forme de tube, soit en une matière élastique au moins transversalement.

L'invention n'est donc pas limitée aux modes de réalisation, non plus qu'au mode d'application, qui ont été décrits ; on pourrait au contraire concevoir diverses variantes sans sortir pour autant de son cadre.

Dans cette demande de brevet, les termes supérieur(e) et inférieur(e) sont définis par le positionnement de l'article lorsqu'il s'applique à l'utilisateur, la partie supérieure étant la partie destinée à s'appliquer sur le membre à contenir du côté de la tête de l'utilisateur et la partie inférieure étant la partie destinée à s'appliquer du côté des pieds de l'utilisateur.

## Revendications

1. Genouillère comportant un panneau (1) en matière souple, ayant une partie supérieure destinée à s'appliquer en partie autour de la partie distale du fémur et une partie inférieure destinée à s'appliquer autour de la partie proximale du tibia, au moins un élément (20, 22, 23, 26, 27) de serrage, notamment au moins une sangle, du panneau au fémur et au tibia, et au moins un montant (18) d'immobilisation en une matière plus rigide que ladite matière souple, **caractérisée en ce que** seule la partie inférieure du panneau (1) comporte du matériau anti-glissement, le matériau anti-glissement est fixé à la partie inférieure sous la forme d'une pièce rapportée et le matériau anti-glissement appartient au groupe constitué par du caoutchouc, naturel ou synthétique, et de la silicone du type anti-glissement.

2. Genouillère, suivant la revendication 1, **caractérisée en ce que** la partie inférieure comprend du matériau anti-glissement au niveau de l'arête tibiale avant.

3. Genouillère suivant l'une des revendications 1 ou 2, **caractérisée en ce que** ledit au moins un montant s'étend du bord de la partie supérieure du panneau au bord de la partie inférieure du panneau.

4. Genouillère suivant l'une des revendication 1 à 3, **caractérisée en ce que** la matière anti-glissement est pleine ou ajourée.

5. Genouillère suivant l'une des revendications 1 à 4, **caractérisée en ce que** le panneau est en une matière élastique au moins transversalement.

6. Genouillère suivant l'une des revendications 1 à 4, **caractérisée en ce que** le panneau est en une matière non élastique, et les moyens de serrage comportent des sangles (20, 22, 26), notamment du type auto-agrippant.

7. Genouillère suivant l'une des revendications 1 à 6, **caractérisée en ce que** la pièce rapportée (31) est amovible, notamment à l'aide d'une fermeture (35, 39) du type auto-agrippant.

8. Genouillère suivant l'une des revendications précédentes, **caractérisée en ce qu'**il est prévu au moins un montant d'immobilisation central, un montant d'immobilisation gauche et un montant d'immobilisation droit.

9. Genouillère suivant la revendication 8, **caractérisée en ce que** les montants d'immobilisation gauche et droit sont inclinés par rapport au montant central.

10. Genouillère suivant l'une des revendications précédentes, **caractérisé en ce que** le panneau a la forme générale d'un trapèze isocèle, la partie supérieure étant de plus grande dimension en largeur que la partie inférieure.

## Claims

1. Knee support comprising a panel (1) made of flexible material, having an upper section intended to be applied in part around the distal portion of the femur and a lower section intended to be applied around the proximal portion of the tibia, at least one element for fixing (20, 22, 23, 26, 27), particularly at least one strap, of the panel to the femur and to the tibia, and at least one immobilising support element (18) made of a more rigid material than said flexible material, **characterised in that** only the lower section of the panel (1) comprises anti-slip material, the anti-slip material is fixed to the lower section in the form of a detachable element and the anti-slip material belongs to the group comprising rubber, natural or synthetic, and silicone of the anti-slip type.

2. Knee support according to claim 1, **characterised in that** the lower section comprises anti-slip material in the area of the front edge of the tibia.

3. Knee support according to one of the claims 1 or 2, **characterised in that** said at least one support element extends from the edge of the upper section of the panel to the edge of the lower section of the panel.

4. Knee support according to one of the claims 1 to 3, **characterised in that** the anti-slip material is complete or perforated.

5. Knee support according to one of the claims 1 to 3, **characterised in that** the panel is made of a material which is elastic at least transversally.

6. Knee support according to one of the claims 1 to 4, **characterised in that** the panel is made of a non-elastic material and the fixing means comprise straps (20, 22, 26), particularly of the hook and loop type.

7. Knee support according to one of the claims 1 to 6, **characterised in that** the detachable element (31) is removable, particularly with the aid of a fastening (35, 39) of the hook and loop type.

8. Knee support according to one of the preceding claims, **characterised in that** at least a central immobilising support element, a left immobilising support element and a right immobilising support element are provided.

9. Knee support according to claim 8, **characterised in that** the left and right vertical immobilising elements are inclined in relation to the central support element.

10. Knee support according to one of the preceding claims, **characterised in that** the panel has the general shape of an isosceles trapezium, the upper section having a greater width measurement than the lower section.

## Patentansprüche

1. Knieschoner, umfassend eine Platte (1) aus elastischem Material mit einem oberen Abschnitt, der dazu bestimmt ist, sich teilweise um den distalen Teil des Femurs herum zu legen, und einem unteren Abschnitt, der dazu bestimmt ist, sich um den proximalen Teil der Tibia herum zu legen, mindestens ein Element (20,22,23,26,27), insbesondere mindestens einen Gurt zum Festziehen der Platte am Femur und an der Tibia, und mindestens ein Feststell-Stützteil (18) aus einem steiferen Material als das elastische Material, **dadurch gekennzeichnet, dass** nur der untere Abschnitt der Platte (1) rutschfestes Material aufweist, wobei das rutschfeste Material an dem unteren Abschnitt in Form eines Einsatzstücks befestigt ist, und das rutschfeste Material der Gruppe angehört, die natürlichen oder synthetischen Gummi sowie Silikon vom rutschfesten Typ umfasst.

2. Knieschoner nach Anspruch 1, **dadurch gekennzeichnet, dass** der untere Abschnitt rutschfestes Material auf Höhe der vorderen Tibia-Kante aufweist.

3. Knieschoner nach einem der Ansprüche 1 oder 2, **dadurch gekennzeichnet, dass** mindestens ein Stützteil sich von dem Rand des oberen Abschnitts der Platte bis zum Rand des unteren Abschnitts der Platte erstreckt.

4. Knieschoner nach einem der Ansprüche 1 bis 3, **dadurch gekennzeichnet, dass** das rutschfeste Material massiv oder durchlässig bzw. durchlöchert ist.

5. Knieschoner nach einem der Ansprüche 1 bis 4, **dadurch gekennzeichnet, dass** die Platte aus zumindest transversal elastischem Material besteht.

6. Knieschoner nach einem der Ansprüche 1 bis 4, **dadurch gekennzeichnet, dass** die Platte aus nicht-elastischem Material besteht, und die Festziehmittel Gurte (20,22,26), insbesondere vom Selbstverschluss-Typ, umfassen.

7. Knieschoner nach einem der Ansprüche 1 bis 6, **dadurch gekennzeichnet, dass** das Einsatzstück (31) abnehmbar ist, insbesondere mit Hilfe eines Verschlusses (35,39) vom Selbstverschluss-Typ.

8. Knieschoner nach einem der vorangehenden Ansprüche, **dadurch gekennzeichnet, dass** mindestens ein zentrales Feststell-Stützteil, ein linkes Feststell-Stützteil und ein rechtes Feststell-Stützteil vorgesehen ist.

9. Knieschoner nach Anspruch 8, **dadurch gekennzeichnet, dass** die linken und rechten Feststell-Stützteile in Bezug auf das zentrale Stützteil schräggestellt sind.

10. Knieschoner nach einem der vorangehenden Ansprüche, **dadurch gekennzeichnet, dass** die Platte allgemein die Form eines gleichschenkligen Trapezes aufweist, wobei der obere Abschnitt eine größere Breitendimension hat als der untere Abschnitt.
